# EUROPÄISCHE PATENTSCHRIFT

(11) **EP 1 416 274 B1**
(45) Veröffentlichungstag und Bekanntmachung des Hinweises auf die Patenterteilung: **21.05.2008**
(21) Anmeldenummer: 03024609.4
(22) Anmeldetag: 25.10.2003
(51) Int. Cl.: G01N 33/52

(54) **Testelementanalysesystem**
Test device system
Système d'analyse

(30) Priorität: 29.10.2002 DE 10250331
(43) Veröffentlichungstag der Anmeldung: 06.05.2004
(73) Patentinhaber: Roche Diagnostics GmbH, 68305 Mannheim (DE); F. Hoffmann-La Roche AG, 4070 Basel (CH)
(72) Erfinder: Frank, Martin, 67246 Dirmstein (DE); Deck, Frank, 67150 Niederkirchen (DE); Fritz, Michael, 68647 Biblis (DE)
(74) Vertreter: Pfeifer, Hans-Peter

(56) Entgegenhaltungen:
- EP-A- 0 285 851
- EP-A- 0 654 668
- US-A- 5 173 261
- US-A- 5 332 549
- US-B1- 6 176 119

## Beschreibung

Die Erfindung betrifft ein Testelementanalysesystem zur analytischen Untersuchung einer flüssigen Probe sowie Testelemente für ein solches Analysesystem und ein Verfahren zu deren Herstellung.

Zur qualitativen und quantitativen Analyse von Bestandteilen einer flüssigen Probe, insbesondere einer Körperflüssigkeit von Menschen oder Tieren, werden in großem Umfang Testverfahren eingesetzt, die mit Testelementen arbeiten. Die Testelemente enthalten Reagenzien. Zur Durchführung einer Reaktion wird das Testelement mit der Probe in Kontakt gebracht. Die Reaktion von Probe und Reagenz führt zu einer für die Analyse charakteristischen Veränderung des Testelementes, die mit Hilfe eines geeigneten Auswertegerätes ausgewertet wird. Das Auswertegerät ist in der Regel zur Auswertung eines ganz bestimmten Typs von Testelementen eines bestimmten Herstellers geeignet. Die Testelemente und das Auswertegerät bilden wechselseitig aufeinander abgestimmte Bestandteile und werden insgesamt als Analysesystem bezeichnet.

Es sind zahlreiche unterschiedliche Testelement-Typen bekannt, die sich durch das Meßprinzip und die verwendeten Reagenzien sowie durch ihren Aufbau unterscheiden.

Hinsichtlich des Meßprinzips sind colorimetrische Analysesysteme besonders weit verbreitet. Bei Ihnen führt die Reaktion der Probe mit den in dem Testelement enthaltenden Reagenzien zu einer Farbänderung, die visuell oder mittels einer photometrischen Meßeinrichtung gemessen werden kann. Daneben haben elektrochemische Analysesysteme eine große Bedeutung erlangt, bei denen die Reaktion der Probe mit den Reagenzien des Testelementes zu einer elektrisch meßbaren Änderung (einer elektrischen Spannung oder eines elektrischen Stromes) führt, die mit einer entsprechenden Meßelektronik gemessen wird.

Hinsichtlich des Aufbaus sind insbesondere streifenförmige Testelemente (Teststreifen) gebräuchlich, die im wesentlichen aus einer länglichen Tragschicht aus Kunststoffmaterial und darauf angebrachten Testfeldern bestehen. Die Testfelder bestehen in der Regel aus einer oder mehreren Reagenzien enthaltenden Testschichten. Solche Teststreifen werden in großem Umfang insbesondere für Blut- und Urinuntersuchungen verwendet.

Bei einem zweiten Typ von Testelementen, der bisher nur in geringem Umfang praktisch angewendet wird, ist ein Testfeld ähnlich wie ein photographisches Diapositiv von einem Rahmen umgeben. Solche Testelemente werden als "Testträger mit Rahmen" und in der englischsprachigen Fachliteratur als "analysis slides" bezeichnet. Das Testfeld dieses Testelementtyps besteht in der Regel aus einer oder mehreren Testschichten, die von dem Rahmen gehalten werden und für colorimetrische Tests geeignete Reagenzien enthalten. Nach Aufgabe der Probe auf das Testfeld und Ablauf der Testreaktion kann die Farbbildung, meist auf der gegenüberliegenden Seite (also in der Regel der Unterseite) des Testfeldes, beobachtet bzw. photometrisch vermessen werden. Derartige Testelemente mit Rahmen sind beispielsweise aus dem US Patent 5,173,261 bekannt.

Daneben sind für spezielle Anwendungsfälle Sonderformen von Testelementen vorgeschlagen worden. Beispielsweise wird in der EP 0312394 A2 ein Testelement für Immuntests beschrieben, bei dem eine Membran, die immunchemische Reagenzien enthält, in einem kegelstumpfförmigen Kunststoffteil eingespannt ist. Das Kunststoffteil wird auf eine Spritze aufgesetzt, um mittels des Spritzenkolbens eine Flüssigkeit durch die Membran zu saugen und dadurch die bei diesem Testtyp erforderliche Trennung gebundener und freier Reagenzbestandteile zu ermöglichen.

Meist werden Testelement-Analysen manuell durchgeführt. Es sind jedoch auch bereits zahlreiche Analysesysteme vorgeschlagen worden, bei denen die Analyse teilweise oder vollständig automatisch abläuft. Die Auswertegeräte solcher Systeme enthalten in der Regel folgende Baugruppen:
- einen Testelement-Vorratsbehälter, in dem eine Mehrzahl von Testelementen vorrätig gehalten wird,
- eine Probenaufgabeeinrichtung, an der das Testelement mit der Probe in Kontakt gebracht wird,
- eine Meßeinrichtung, mit der an einem in einer Meßposition (die mit der Probenaufgabeposition übereinstimmen oder von dieser verschieden sein kann) befindlichen Testelement eine für die Analyse charakteristische Meßgröße gemessen wird, und
- eine Transporteinrichtung, durch die je ein Testelement aus dem Testelement-Vorratsbehälter entnommen, zu der Probenaufgabeeinrichtung transportiert und erforderlichenfalls nach Kontaktierung mit der Probe zu der Meßposition weitertransportiert wird.

Zur konstruktiven Gestaltung wurden ganz unterschiedliche Vorschläge gemacht. Beispielsweise sind in den US-Patenten 3,932,133 und 4,876,204 Auswertegeräte beschrieben, bei denen der Testelement-Vorratsbehälter als Magazin ausgebildet ist, in dem eine Mehrzahl von Teststreifen übereinander gestapelt vorrätig gehalten werden. Um sie von dort zu entnehmen und zu den nachfolgenden Bearbeitungsstationen zu transportieren, ist eine Transporteinrichtung mit einem Greifer vorgesehen, der jeweils einen Teststreifen ergreift.

In der GB 2014113 A der EP 0054849 und dem US-Patent 5,143,694 werden unterschiedliche Entwicklungen beschrieben, denen gemeinsam ist, daß Teststreifen mittels einer Transporteinrichtung kontinuierlich quer zu ihrer Längsrichtung an den erforderlichen Bearbeitungsstationen vorbei transportiert werden. Hierzu dient bei der GB 2014113 eine Zylinderwalze, bei der EP 0054849 ein kontinuierlich transportierter Papierstreifen und bei dem US-Patent 5,143,694 ein System von Transportfingern, die die Teststreifen über in einem Kunststoffeinsatz ausgebildeten Schienen von einer Aufgabeposition über eine Meßstation bis zu einem Abfallbehälter schieben.

Alle diese automatisch arbeitenden Testelementanalysesysteme haben einen hohen Platzbedarf. Sie sind konstruktiv aufwendig und benötigen relativ viel elektrische Energie. Deshalb eignen sie sich nicht für kleine tragbare batteriebetriebene Analysesysteme, wie sie insbesondere für die Blutzucker-Selbstkontrolle ("home monitoring") von Diabetikern gebräuchlich sind.

Um auf dem Gebiet der Blutzucker-Selbstkontrolle eine vereinfachte Handhabung zu erreichen, wird in der EP 0823635 eine spezielle Form von Testelementen vorgeschlagen, bei denen das Testfeld in die Stirnfläche einer Halterung integriert ist, die kegelstumpfförmig oder pyramidenstumpfförmig ausgebildet ist. Eine Mehrzahl der Testelemente wird in einem röhrenförmigen Magazin übereinander gestapelt und derartig bereitgehalten, daß ein entsprechend ausgebildetes Auswertegerät jeweils auf das oberste Testelement in dem Magazin gesteckt werden kann. Dabei schnappt ein Vorsprung des Testelementes in einer entsprechenden Vertiefung am Kopf des Auswertegerätes ein, so daß eine Verbindung zwischen dem Testelement und dem Auswertegerät hergestellt wird. Anschließend wird das an dem Gerät befestigte Testelement mit einem Blutstropfen in Kontakt gebracht, der beispielsweise an einer Fingerspitze erzeugt wurde. Die aufwendige Form dieser Testelemente verursacht erhebliche Kosten. Dennoch wird die Handhabung gegenüber konventionellen Teststreifen nicht wesentlich vereinfacht.

In der EP 0922959 ist ein Analysesystem beschrieben, das einen feuchtigkeitsdichten Vorratsbehälter für Testelemente und ein Auswertegerät umfaßt. Das Auswertegerät ist mit zwei Führungsnuten versehen, nämlich einer Führungsnut, die auf eine entsprechende Profilierung der Testelemente abgestimmt ist und eine zweite Führungsnut, die mit einem entsprechenden Führungselement des Vorratsbehälters zusammenwirkt. Um ein Testelement aus dem Vorratsbehälter zu entnehmen und in der Testelement-Halterung des Auswertegerätes zu plazieren, werden beide Komponenten zusammengesteckt, wobei eine direkte Übernahme eines Testelementes aus dem Vorratsbehälter in das Gerät stattfindet. Dadurch wird die Handhabung erleichtert, jedoch ist die Konstruktion relativ aufwendig und der Materialverbrauch für die Herstellung der Testelemente ist verhältnismäßig hoch.

Weitere Testelementanalysesysteme, bei denen ein mit einem Rahmen versehenes Testelement verwendet wird, sind in den Dokumenten EP 0452740A und DE 10101658A beschrieben.

Der Erfindung liegt auf dieser Basis die Aufgabe zugrunde, ein Testelementanalysesystem zu schaffen, bei dem eine Verbesserung der Handhabung, insbesondere hinsichtlich der Entnahme der Testelemente aus einem Vorratsbehälter und ihrer Zuführung zu einer Probenaufgabeposition des Auswertegerätes mit geringem Aufwand erreicht wird. Die Konstruktion soll einfach sein und sich auch für kleine, tragbare, batteriebetriebene Analysesysteme eignen.

Diese Aufgabe wird gelöst durch ein Testelementanalysesystem nach Anspruch 1 und durch ein Testelement nach Anspruch 13.

Die Erfindung ist sowohl für colorimetrische als auch für elektrochemische Analysesysteme geeignet. Als Testfeld wird hier derjenige Bereich des Testelementes bezeichnet, der mit der Probe in Kontakt gebracht und dabei von der Probe benetzt wird. Der Rahmen, der das Testfeld umgibt, muß nicht von einem gesonderten Bauteil gebildet werden. Insbesondere bei elektrochemischen Testelementen ist es zweckmäßig, wenn das Testfeld und der Rahmen einstückig aus einem Kunststoffteil hergestellt sind, wobei auf oder in der Testfeldoberfläche die erforderlichen Elektroden integriert sind. In jedem Fall wird der Rahmen, der das Testfeld zumindest teilweise umgibt, nicht von der Probe benetzt und weist den am äußeren Umfang des Testelementes umlaufenden Greifrand auf.

Durch die Erfindung werden wesentliche Vorteile erreicht:
- Die Testelemente können einfach und kostengünstig hergestellt werden. Der zweckmäßigerweise aus Metall oder einem Kunststoffmaterial (das bevorzugt ein Polycarbonat oder einen Polyester enthält) hergestellte Rahmen kann sehr dünn und schmal gestaltet werden. Dadurch wird nicht nur der Materialverbrauch, sondern auch das Verpackungsvolumen der Testelemente reduziert, d.h. eine große Zahl von Testelementen kann in einem Magazin mit relativ geringem Volumen vorrätig gehalten werden. Entsprechend wird auch der Raumbedarf für einen zur Aufnahme verbrauchter Testelemente geeigneten Abfallbehälter reduziert.
- Die Transporteinrichtung automatischer Auswertegeräte kann mit einem Minimum beweglicher Teile sehr einfach konstruiert werden.
- Auch die teilweise Mechanisierung der Handhabungsschritte durch Verwendung eines Systems, das als funktional aufeinander abgestimmte Elemente einen speziellen Vorratsbehälter und ein entsprechendes Auswertegerät (wie bei der erwähnten EP 0922959) umfaßt, ist mit geringerem Aufwand möglich.
- Zugleich wird eine zuverlässige Funktion erreicht. Dies gilt in besonderem Maße, wenn das Querschnittsprofil des Rahmens im Bereich des Greifrandes so gestaltet ist, daß die Verbindung zwischen den Greifarmen und dem Testelement nicht nur auf Kraftschluß, sondern auf Formschluß basiert. Konkret wird dies dadurch erreicht, daß der Durchmesser des Rahmens von der Greiffläche aus in mindestens einer senkrecht zu der Testfeldebene verlaufenden Richtung unter Ausbildung einer vorspringenden Schulter zunimmt.

Die Erfindung wird nachfolgend anhand von in den Figuren schematisch dargestellten Ausführungsformen näher erläutert. Die beschriebenen Besonderheiten können einzeln oder in Kombination verwendet werden, um bevorzugte Ausgestaltungen der Erfindung zu schaffen. Es zeigen:
- Fig. 1: eine perspektivische Prinzipsskizze der Bewegung einer Greifeinrichtung zum Transport eines Testelementes zwischen drei Funktionspositionen eines Auswertegerätes.
- Fig. 2: eine perspektivische, teilweise aufgeschnittene Darstellung einer alternativen Ausführungsform eines Testelementes.
- Fig. 3: eine perspektivische, teilweise aufgeschnittene Darstellung eines Stapels der Testelemente gemäß Figur 2.
- Fig. 4: eine Detaildarstellung des Ausschnitts A aus Figur 3.
- Fig. 5: eine perspektivische Darstellung eines Greifarmes bei der Entnahme eines Testelementes gemäß den Figuren 2 bis 4 aus einem röhrenförmigen Magazin.
- Fig. 6: eine perspektivische Darstellung von zwei Bewegungsphasen (a) und (b) beim Ergreifen eines Testelementes mittels eines Greifarms.
- Fig. 7: eine Darstellung entsprechend Figur 2 von einer weiteren alternativen Ausführungsform eines Testelementes.
- Fig. 8: eine teilweise aufgeschnittene Darstellung des Testelementes gemäß Figur 7 mit eingesetztem und befestigtem Testfeld.
- Fig. 9: eine Detaildarstellung des Ausschnitts B aus Figur 8.
- Fig. 10: einen Querschnitt durch den Rahmen einer weiteren alternativen Ausführungsform eines Testelementes.
- Fig. 11: eine Detaildarstellung des Ausschnitts C aus Figur 10.
- Fig. 12: eine perspektivische Darstellung einer alternativen Ausführungsform einer Greifeinrichtung mit einem noch nicht ergriffenen Testelement.
- Fig. 13: eine perspektivische Darstellung der Greifeinrichtung gemäß Figur 11 mit ergriffenem Testelement.
- Fig. 14: eine Detaildarstellung zur Verdeutlichung des Zusammenwirkens der Greifarme und des Greifrandes bei der Ausführungsform der Figuren 12 und 13,
- Fig. 15: eine perspektivische Darstellung eines teilmechanisierten Analysesystems,
- Fig. 16: eine perspektivische, teilweise aufgeschnittene Darstellung des Systems nach Figur 15 während der Übernahme eines Testelementes aus einem Magazin in das Auswertegerät, und
- Fig. 17: eine Detaildarstellung zu Figur 16.

Die in den Figuren dargestellten Testelemente 1 weisen jeweils einen eine Testfeldöffnung 2 umgebenden Rahmen 3 und ein in der Testfeldöffnung 2 angeordnetes Testfeld 5 auf. Das Testfeld 5 definiert eine (in den Figuren durch zwei Geraden 4a und 4b bezeichnete) Testfeldebene.

Bei sämtlichen in den Figuren dargestellten Ausführungsformen besteht das Testfeld aus einer einzigen Schicht bzw. einem Schichtverbund mehrerer miteinander fest verbundener (in der Regel unterschiedliche Reagenzien enthaltender) Schichten. Die Erfindung kann grundsätzlich jedoch auch in Verbindung mit Testfeldern verwendet werden, die aus mehreren lose aufeinander liegenden Schichten bestehen. Die Mehrzahl der Schichten wird in diesem Fall von dem Rahmen 3 fixiert und zusammengehalten, wie dies beispielsweise in dem US-Patent 5,173,261 beschrieben ist. Wie erwähnt sind, vor allem bei elektrochemischen Testelementen, auch Gestaltungen möglich, bei denen das Testfeld und der Rahmen keine getrennte Teile sind.

Die Testelemente 1 sind scheibenförmig in dem Sinn, daß ihre Dicke d in axialer Richtung Z erheblich kleiner ist als ihre Abmessungen in den hierzu senkrechten Raumrichtungen. Die dargestellte in Aufsicht auf die Testfeldebene kreisrunde (bevorzugt rotationssymmetrische) Form der Testelemente 1 ist zwar bevorzugt, aber nicht unbedingt notwendig. Vielmehr können die Testelemente 1 auch eine von der Kreisform abweichende äußere Form haben. Deshalb ist der Begriff "scheibenförmig" nicht beschränkend im Sinne von "kreisscheibenförmig" zu verstehen.

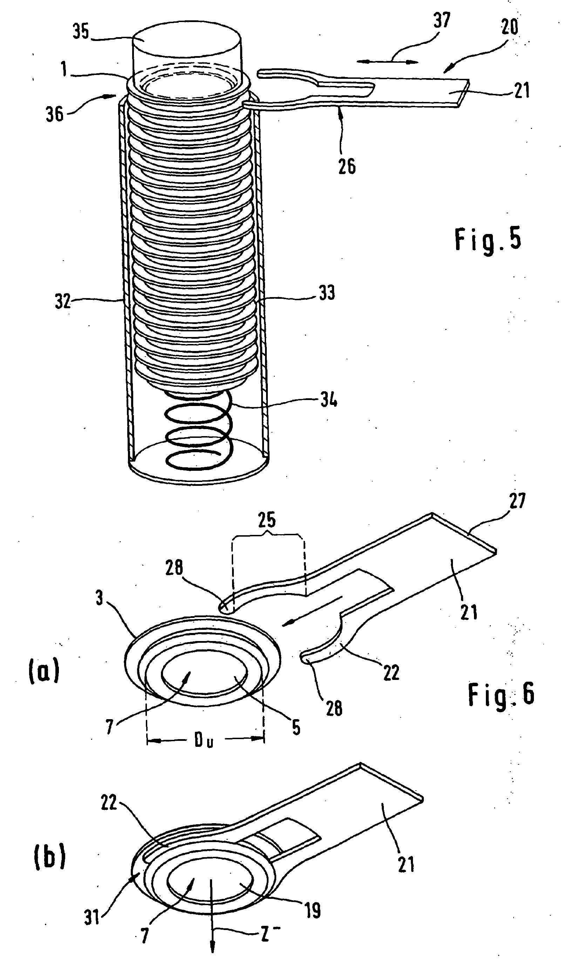

Eine zumindest zentralsymmetrische Gestaltung mit einer gekrümmten Begrenzung (beispielsweise eine elliptische oder ovale Form) ist bevorzugt. Mit einer solchen, vorzugsweise kreisrunden, Geometrie sind eine Reihe erheblicher Vorteile verbunden: Die Halterung von runden Testelementen in der Greifeinrichtung ist selbstzentrierend, die Konstruktion des Vorratsbehälters in Form eines Magazins (Figur 5) ist einfacher und die Relation zwischen der Größe der Probenaufgabefläche 18 und der Gesamtfläche des Testelementes 1 ist besonders günstig. Grundsätzlich ist aber auch eine rechteckige oder sogar nichtsymmetrische Formgebung der Testelemente 1 möglich.

Auch für nicht-rotationssymmetrische Testelemente wird die Z-Richtung senkrecht zu der Testfeldebene (die im Falle eines rotationssymmetrischen Testelementes mit dessen Symmetrieachse zusammenfällt) als "axiale Richtung" bezeichnet, während die Bezeichnung "radial" für eine parallel zu der Testfeldebene vom Zentrum des Testfeldes 5 weg (oder von dessen Rand auf dessen Zentrum zu) verlaufende Raumrichtung verwendet wird.

Die flüssige Probe wird auf einer Probenaufgabenseite 6 des Testelementes 1 aufgegeben. Sie durchdringt das Testfeld und reagiert mit in dem Testfeld enthaltenen Reagenzien. Die Reaktion führt bei dem dargestellten colorimetrischen Test zu einer für die Analyse charakteristischen, photometrisch meßbaren Farbänderung in einer Auswertezone des Testfeldes auf der Auswerteseite 7 des Testelementes 1, die der Probenaufgabenseite 6 gegenüberliegt.

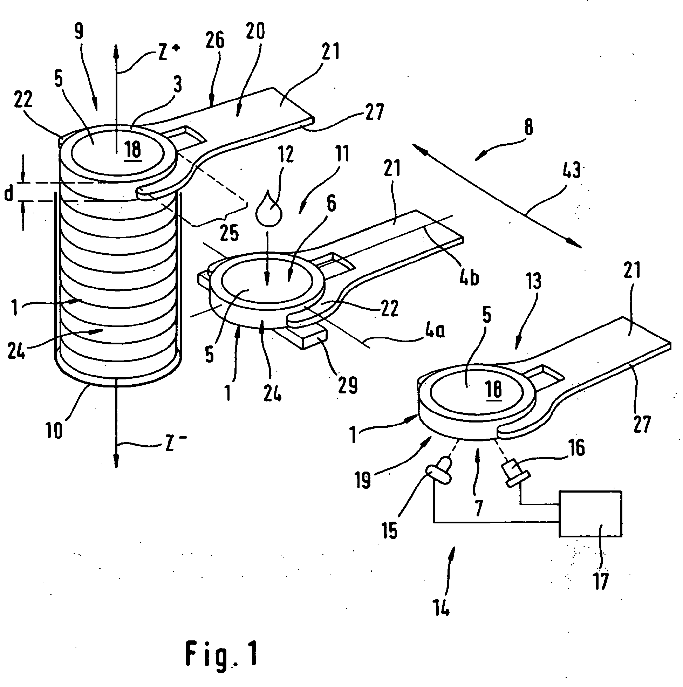

Innerhalb des in Figur 1 nicht in seiner Gesamtheit dargestellten Auswertegerätes werden die Testelemente 1 mittels einer Transporteinrichtung 8 zwischen mehreren Funktionspositionen bewegt. In Figur 1 sind drei Funktionspositionen dargestellt, nämlich eine Entnahmeposition 9, an der ein Testelement aus einem vorzugsweise als Magazin ausgebildeten (hier nur schematisch angedeuteten) Vorratsbehälter 10 entnommen wird, eine Probenaufgabeposition 11, an der das Testelement 1 mit der Probe 12 in Kontakt gebracht wird, und eine Meßposition 13, an der mittels einer Meßeinrichtung 14 eine für die Analyse charakteristische Meßgröße gemessen wird. Im dargestellten Fall handelt es sich um eine reflexionsphotometrische Meßeinrichtung mit einem Lichtsender 15, einem Lichtempfänger 16 und einer Meß- und Auswerteelektronik 17. Damit wird in bekannter Weise die Remission der (im dargestellten Fall von der Unterseite des Testfeldes 5 gebildeten) Auswertezone 19 gemessen.

Die Transporteinrichtung 8 ergreift und transportiert jeweils ein Testelement 1 mittels einer Greifeinrichtung 20, die hier als Greifgabel 21 mit zwei Armen 22 ausgebildet ist. Der Bereich des Rahmens, an dem er von den Armen kontaktiert wird, wird als Greifrand 24 bezeichnet. Er wird von einer radial nach außen, d.h. vom Zentrum des Testfeldes 5 weg, gerichteten, am äußeren Umfang des Rahmens 3 zumindest abschnittsweise umlaufenden Randfläche gebildet. Der Abschnitt der Arme 22, auf dem der Kontakt zu dem Greifrand 24 stattfindet, wird als Greifabschnitt 25 bezeichnet. In dem Greifabschnitt 25 können die Greifarme 22 entweder vollflächig oder punktweise in Kontakt zu dem Greifrand 24 des Rahmens 3 stehen. Der Greifabschnitt 25 ist der zwischen dem ersten und letzten Kontaktpunkt liegende Teil der Arme 22. Er verläuft parallel zu der Testfeldebene 4.

Durch eine als Greifgabel 21 ausgebildete Greifeinrichtung 20 wird eine besonders platzsparende und zuverlässige Konstruktion erreicht, wobei es möglich ist, den gesamten Transport der Testelemente 1 zwischen den Funktionspositionen des Auswertegerätes mittels einer einfachen Schwenkbewegung der Greifgabel 21 um eine feste Achse zu realisieren.

Im Gegensatz zu den bei bekannten automatischen Testelementanalysesystemen verwendeten Greifern kontaktiert die Greifeinrichtung 21 nicht die räumlich ausgedehnte Oberund Unterseite einer Tragschicht, sondern einen schmalen Greifrand von außen her. Da die Haltekraft in radialer Richtung von außen nach innen gerichtet ist, kann die Greifeinrichtung 20 auch als Radialgreifer bezeichnet werden.

Die Dicke der Testelemente 1 (d.h. ihre maximale Dimension in axialer Richtung) beträgt vorzugsweise weniger als 3 mm. Um ein sicheres Festhalten mittels des schmalen Greifrandes 24 zu gewährleisten, sollte sie mindestens 0,3 mm betragen. Vorzugsweise liegt die Dicke bei 0,5 mm bis 1 mm.

Die Rahmenfläche (Flächenausdehnung des Rahmens, betrachtet in Aufsicht auf die Testfeldebene) ist bei der dargestellten bevorzugten Ausführungsform kleiner, zumindest nicht viel größer als die Probenaufgabefläche 18 des Testfeldes 5. Vorzugsweise ist die Rahmenfläche höchstens dreimal so groß wie die Probenaufgabefläche 18 des Testfeldes. Testelemente 1 mit einem schmalen Rahmen zeichnen sich durch einen geringen Materialverbrauch aus und können sehr platzsparend gelagert werden. Das wird durch die insgesamt kleinen Abmessungen erfindungsgemäßer Testelemente zusätzlich unterstützt. Vorzugsweise ist die größte Dimension der Testelemente in radialer Richtung (bei runden Testelementen deren Durchmesser) kleiner als 10 mm, bevorzugt kleiner als 6 mm. In der praktischen Erprobung der Erfindung wurden Analyseelemente mit nur etwa 4 mm Außendurchmesser und einem Durchmesser der Probenaufgabefläche 18 von 3 mm (d.h. einer Randbreite von 0,5 mm) eingesetzt.

Grundsätzlich können die Greifarme 22 über jeweils ein Schwenklager mit dem übrigen Teil der Greifeinrichtung 20 verbunden und mechanisch beweglich sein. Bevorzugt ist jedoch eine Ausführungsform, bei der die zum Greifen und Festhalten der Testelemente 1 erforderliche Beweglichkeit darauf beruht, daß die Arme 22 derartig elastisch mit der übrigen Greifeinrichtung 20 verbunden sind, daß sie allein aufgrund dieser Elastizität (also ohne daß die Arme 22 der Greifgabel 21 durch eine Betätigungseinrichtung bewegt werden müssen) dergestalt auf bzw. um das Testelement geschoben werden können, daß sie das Testelement festhalten. Bevorzugt resultiert die elastische Beweglichkeit aus einer Eigenelastizität des hier von der Greifgabel 21 gebildeten Greifelementes 26, d.h. das Greifelement 26 einschließlich der Arme 22 ist bezüglich einer Bewegung parallel zu der Testfeldebene elastisch verformbar und so ausgebildet, daß es unter Biegespannung steht, wenn ein Testelement 1 festgehalten wird. Vorzugsweise sind, wie dargestellt, die Arme 22 der Greifgabel 21 Bestandteil eines einstückig aus einem elastischen Material (Metall oder Kunststoff), beispielsweise durch Pressen oder Stanzen, hergestellten Gabelteils 27.

Um ein sicheres Festhalten der Testelemente 1 zu fördern, sind die Greifarme 22 vorzugsweise - wie dargestellt - so gestaltet, daß ihr Abstand zum vorderen Ende 28 des Greifabschnitts 25 hin abnimmt. Der Greifrand 24 eines runden Testelementes 1 wird dabei von den Armen 22 um mehr als 180 Grad umgriffen. Dadurch wird die Fixierung des Testelementes 1 insbesondere gegen ein Herausrutschen infolge einer zu der Testfeldebene parallelen Bewegung verbessert.

Bei der in Figur 1 dargestellten Ausführungsform wird der Greifrand 24 von einer in axialer Richtung Z gerade verlaufenden Zylindermantelfläche gebildet. Das Testelement wird hinsichtlich einer Verschiebung in Z-Richtung nur durch Kraftschluß fixiert. Die dabei mögliche Haltekraft kann unter Umständen unzureichend sein, beispielsweise wenn in der Probenaufgabeposition 11 ein Blutstropfen aufgegeben wird und der Benutzer dabei (versehentlich) gegen das Testelement 1 drückt. Um in einem solchen Fall ein Herausfallen des Testelementes 1 aus der Greifgabel 21 zu vermeiden, kann eine zusätzliche geräteseitige Abstützung 29 vorgesehen sein, auf der das Testelement 1 aufliegt, wenn es sich in der Probenaufgabeposition 11 befindet.

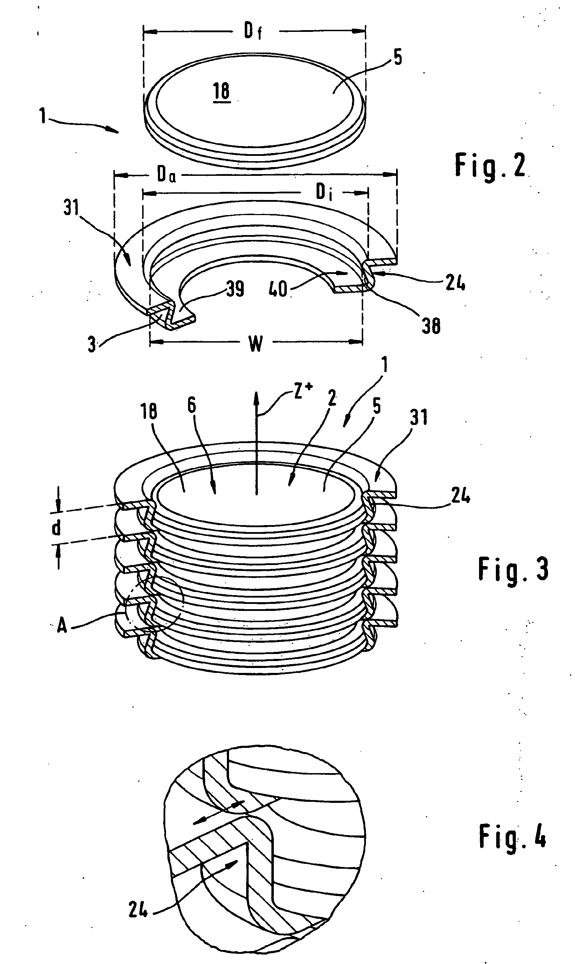

In den Figuren 2 bis 11 sind bevorzugte Ausführungsformen dargestellt, bei denen durch die Gestaltung des Profils des Rahmens 3 im Bereich des Greifrandes 24 eine verbesserte Fixierung der Testelemente 1 an der Greifeinrichtung 20 erreicht wird. Gemeinsam ist diesen Ausführungsformen, daß der Durchmesser des Rahmens 3 jeweils von dem Greifrand 24 aus in beide senkrecht zu der Testfeldebene 4a, 4b verlaufende Richtungen Z⁺ und Z⁻(also bei der in den Figuren dargestellten horizontalen Lage der Testelemente 1 nach oben und unten) zunimmt. Der Greifrand befindet sich demzufolge, betrachtet in axialer Richtung Z, an der Stelle des geringsten Durchmessers der radial nach außen gerichteten umlaufenden Begrenzungsfläche des Rahmens 3. Dadurch wird eine formschlüssige Fixierung der Testelemente 1 an der Greifeinrichtung 20 bezüglich beider axialer Raumrichtungen Z⁺ und Z⁻ erreicht.

Eine erste Ausführungsform solcher Testelemente 1 ist in den Figuren 2 bis 7 dargestellt. Oberhalb des Greifrandes 24 erweitert sich der Rahmen 3 zu einer flanschartigen Schulter 31. Das Material der Schulter 31 sollte (zumindest an der Oberfläche der Probenaufgabeseite 6) hydrophob (erforderlichenfalls durch Oberflächenbehandlung hydrophobisiert) sein, um die hygienische Wirksamkeit zu optimieren. Die Schulter 31 ist vorzugsweise so breit, daß benachbarte Teile der Greifeinrichtung 20 (hier der Greifabschnitt 25 der an dem Greifrand 24 anliegenden Arme 22) zumindest teilweise, bevorzugt vollständig, von der Schulter 31 abgedeckt werden. Dadurch wird eine Kontamination dieser Teile und damit des Auswertegerätes beim Aufgeben der Probe 12 in der Probenaufgabeposition 11 zuverlässig und mit geringem Aufwand verhindert.

Nach unten hin (in der Richtung Z⁻) nimmt der Durchmesser des Rahmens 3 von dem Greifrand 24 nur relativ geringfügig zu. Diese schwache Profilierung ist ausreichend, um die erforderliche Fixierung des Testelementes 1 gegen ein Herausrutschen nach oben (in Z⁺-Richtung) aus der Greifgabel 21 zu gewährleisten.

Bei der Gestaltung des in den Figuren 2 und 4 besonders deutlich erkennbaren Randprofils des Rahmens 3 ist auch zu berücksichtigen, daß das Querschnittsprofil des Testelementes 1 vorzugsweise so geformt sein sollte, daß mehrere übereinander gestapelte Testelemente 1 gegeneinander in Richtung der Testfeldebene 4a, 4b gleiten können, ohne zu verhaken. Dadurch wird u.a. erreicht, daß die Testelemente 1 auf einfache Weise ohne zusätzliche bewegliche Teile aus einem Magazin 32 herausgezogen oder -geschoben werden können.

Ein solcher Entnahmevorgang ist in Figur 5 schematisch dargestellt. Die Testelemente 1 sind in einem Vorratsbehälter 10 in Form eines röhrenförmigen Magazins 32 unmittelbar übereinander gestapelt. Um sie nacheinander durch die mit dem Pfeil 37 bezeichnete translatorische Entnahmebewegung an der gleichen Entnahmeposition entnehmen zu können, wird der Stapel 33 der Testelemente 1 beispielsweise von einer Feder 34 nach oben gegen ein festes Gegenstück 35 gedrückt. Der zwischen dem Gegenstück 35 und der oberen Begrenzung des Gehäuses des Magazins 32 verbleibende Entnahmeschlitz 36 ist etwas höher als die Dicke d eines Testelementes, so daß jeweils ein Testelement von einer Greifgabel 21 entnommen werden kann.

Um dies zu ermöglichen, müssen die Testelemente 1 im gestapelten Zustand aufeinander gleiten können. Diese Gleitfähigkeit wird durch die dargestellte Profilierung des Rahmens unterstützt, die dadurch gekennzeichnet ist, daß auf der einen Seite (hier der Probenaufgabeseite 6) dessen Innenmaß Dᵢ kleiner, aber dessen Außenmaß Dₐ grö-βer, als das Außenmaß Dᵤ (Fig. 6) auf der gegenüberliegenden Seite (die auf der ersten Seite gleiten soll; hier der Auswerteseite 7) ist (d.h. Dᵢ < Dᵤ < Dₐ). Bei einer solchen Formgebung haben aufeinandergestapelte Testelemente 1 einen ringförmigen mechanischen Kontakt. Durch die resultierende vorteilhafte Druckverteilung wird das Risiko, daß die Testelemente 1 während einer längeren Lagerperiode miteinander verkleben, reduziert.

Die Profilierung des Rahmens 3 ist auch hinsichtlich einer einfachen und zuverlässigen Fixierung des Testfeldes 5 in dem Rahmen 3 von Bedeutung. Bei Verwendung eines getrennt von dem Rahmen 3 hergestellten Testfeldes 5 ist er vorzugsweise so gestaltet, daß der Rahmen 3 eine Aufnahmemulde 40 zur Aufnahme des Testfeldes 5 umschließt und die Tiefe der Aufnahmemulde 40 größer als die Dicke des Testfeldes 5 ist, so daß die umlaufende Begrenzungswand 38 der Aufnahmemulde 40 die Oberfläche eines darin aufgenommenen Testfeldes 5 überragt. Dadurch kommt das Testfeld 5 nicht mit benachbarten Testelementen in einem Testelementstapel in Kontakt.

Bei der in den Figuren 2 bis 7 dargestellten Ausführungsform haben die Begrenzungswände 38 der Aufnahmemulde 40 auf dem unteren Teilabschnitt ihrer Höhendimension eine negative Steigung (d.h. sie sind von unten nach oben gesehen nach innen geneigt), so daß der Durchmesser der Aufnahmemulde 40 an deren Boden 39 größer als oberhalb des Bodens ist. Das Testfeld 5 ist in der Testfeldaufnahmemulde 40 dadurch fixiert, daß deren lichte Weite W (minimale Abmessung in einer Ebene, die zu der Testfeldebene parallel verläuft) etwas kleiner als der Durchmesser D_{f} des Testfeldes ist, so daß das Testfeld beim Einsetzen in die Aufnahmemulde 40 in radialer Richtung leicht komprimiert wird. Das Testfeldmaterial 5 ist ausreichend elastisch, daß es sich nach dem Einsetzen in die Aufnahmemulde 40 wieder ausdehnt und in einem Paßsitz in der Aufnahmemulde 40 fixiert ist.

Vorteilhafterweise ist der Vorratsbehälter 10 möglichst dicht geschlossen, um die Lagerfähigkeit feuchtigkeitsempfindlicher Testelemente zu erhöhen. Bei einem Magazin der in Figur 5 dargestellten Bauart kann eine ausreichende Dichtwirkung durch Anpassung des Innendurchmessers an den Außendurchmesser der Testelemente 1 und durch entsprechende Gestaltung des Gegenstücks 35 erreicht werden. Grundsätzlich kann der Testelement-Vorratsbehälter jedoch auch offen sein, beispielsweise wenn die Testelemente nicht empfindlich auf Umwelteinflüsse reagieren oder sich der Behälter in einem dichten Gerätegehäuse befindet.

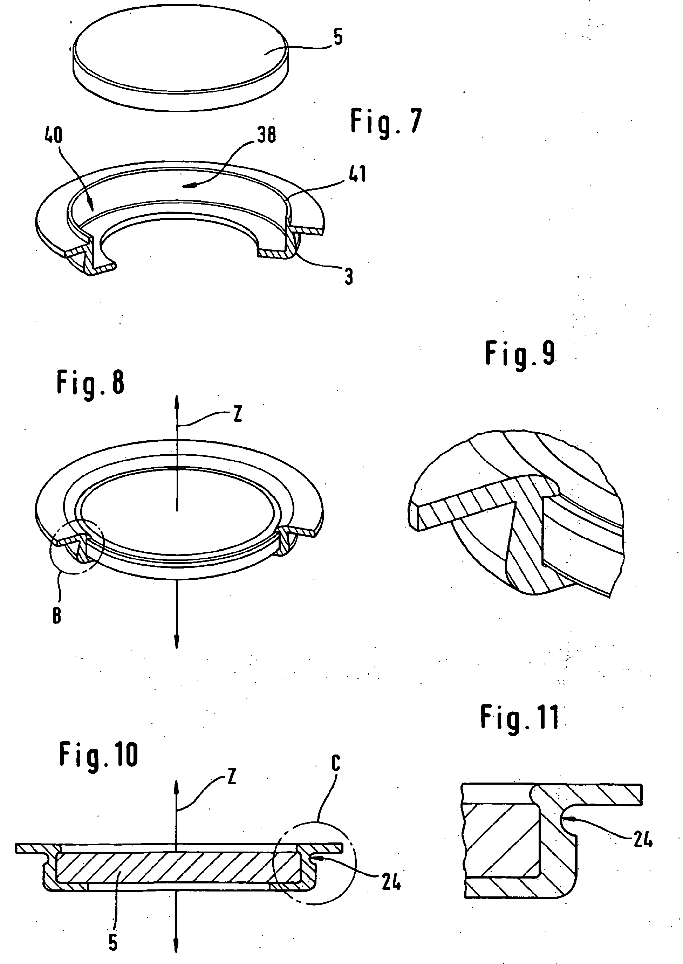

Die Figuren 7 bis 9 zeigen eine Ausführungsform, die sich besonders in solchen Fällen eignet, bei denen das Material des Testfeldes 5 nicht ausreichend elastisch ist, um in der zuvor beschriebenen Weise in dem Rahmen 3 fixiert zu werden. Dabei erfolgt die Fixierung durch Umbördeln eines am Rand der Testfeldmulde 40 vorgesehenen Fixierungsgrates 41 aus der in Figur 7 dargestellten Position in die in Figur 8 dargestellte Position. Dies setzt voraus, daß der Rahmen 3 aus einem plastisch verformbaren Material wie beispielsweise Metall besteht. Es können jedoch auch Kunststoffe auf diese Weise verarbeitet werden.

Bei der in den Figuren 10 und 11 dargestellten Ausführungsform stimmt das Prinzip der Befestigung des Testfeldes 5 mit den Figuren 2 bis 6 überein. Das Profil unterscheidet sich jedoch im Bereich des Greifrandes 24 insofern, als dieser von einer im Querschnitt konkav gekrümmten nutförmigen Vertiefung gebildet wird.

Ein besonderer Vorteil der dargestellten Ausführungsform der Erfindung besteht darin, daß der Transport zwischen den in dem Auswertegerät erforderlichen Funktionspositionen mit wenigen einfachen mechanischen Bewegungen möglich ist. Das Ergreifen eines Testelementes 1 erfordert nur eine einfache translatorische Relativbewegung (Pfeil 37 in Fig. 5) der Greifgabel 21 auf den Stapel der Testelemente 1 zu (oder alternativ des Stapels der Testelemente 1 auf die Greifgabel 21 zu). Anschließend genügt eine einfache Querbewegung - wiederum parallel zu der Testfeldebene (Pfeil 43 in Fig. 1) - um das Testelement den weiteren Funktionsstationen zuzuführen. Sie kann beispielsweise als Schwenkbewegung der Greifgabel 21 um eine ortsfeste Achse realisiert sein. Damit ist auf sehr einfache Weise die vollständige Automatisierung des Analysevorgangs in einem Auswertegerät auch dann möglich, wenn dieses sehr kompakte Abmessungen hat und batteriebetrieben arbeitet.

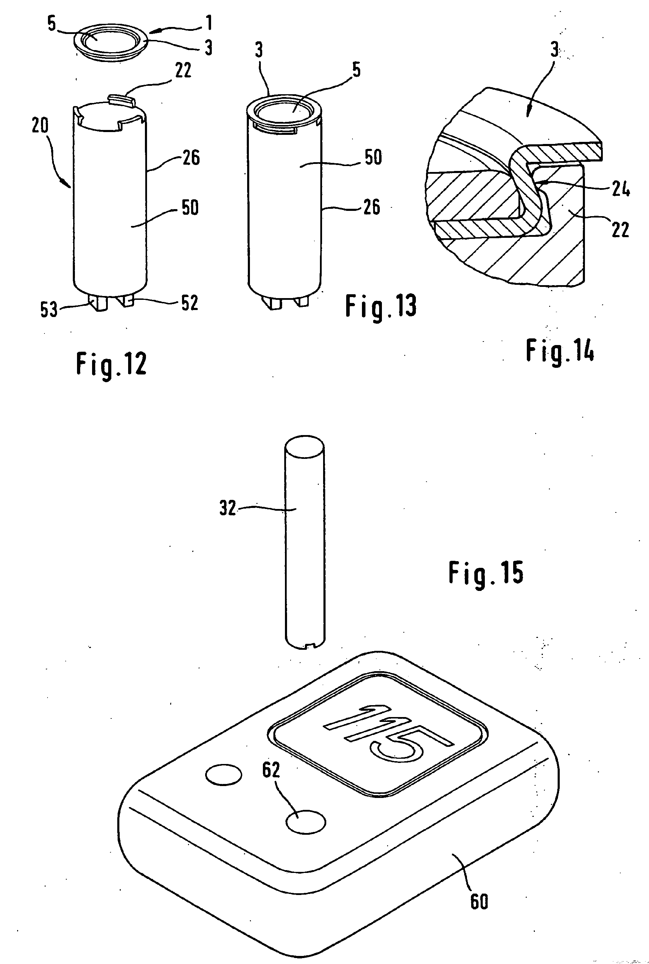

Bei der in den Figuren 12 bis 14 dargestellten Ausführungsform ist das Greifelement 26 der Greifeinrichtung 20 als längliche Greifröhre 50 mit an ihrem oberen Ende hervorstehenden kurzen Armen 22 ausgebildet. Bezogen auf das Testfeld 5 des Testelementes 1 erstrecken sich die Arme 22 senkrecht zu der Testfeldebene (also in Z-Richtung). Auch in diesem Fall beruht die erforderliche Beweglichkeit der Arme 22 auf der Eigenelastizität des hier von der Greifröhre 50 gebildeten Greifelementes 26. Die Arme 22 sind also auch in diesem Fall bezüglich einer Bewegung parallel zu der Testfeldebene derartig elastisch verformbar, daß die Greifröhre durch eine Bewegung in Z-Richtung von unten über den Rahmen 3 des Testelementes 1 geschoben werden kann, wobei die Arme 22 das Testelement 1 durch Kontakt mit dem Greifrand 24 festhalten.

Eine solche Ausführungsform ist für colorimetrische Analysesysteme vorteilhaft, wobei in die Greifröhre zugleich Lichtleiter 52,53 integriert sein können, die zum Ein- und Auskoppeln des Lichts dienen. Im Falle elektrochemischer Analysesysteme können die erforderlichen elektrischen Kontakte sowohl bei dieser Ausführungsform als auch bei den zuvor dargestellten Ausführungsformen in die Greifarme integriert sein. Ein weiterer Vorteil der Ausbildung der Greifeinrichtung 20 als Greifröhre 50 liegt in der für manche Gerätebauformen günstigeren in radialer Richtung platzsparenden Geometrie.

Bei den dargestellten Ausführungsformen steht die Greifeinrichtung 20 jeweils über Greifarme 22 mit dem Greifrand 24 in Kontakt. Es sind jedoch auch Ausführungsformen möglich, bei denen die Greifeinrichtung keine (elastisch gelagerten) Greifarme aufweist, sondern durch Relativverschiebung beweglicher Teile der Greifrand derartig umschlossen wird, daß eine Fixierung durch Formschluß erreicht wird.

Wie bereits erläutert, ist die Erfindung nicht nur bei einem vollautomatischen Analysesystem (wie in Fig. 1 dargestellt), sondern auch bei einem teilmechanisierten Analysesystem vorteilhaft. Dabei kann die erläuterte Formgebung des Testelementes in Verbindung mit einer entsprechenden Greifeinrichtung vorteilhaft verwendet werden, um Testelemente aus einem zu dem System gehörigen Vorratsbehälter (insbesondere Magazin) zu entnehmen und in eine entsprechende Halterung des zugehörigen Auswertegerätes einzusetzen. Besonders bevorzugt ist dabei eine Ausführungsform, bei der (insoweit übereinstimmend mit der EP 0922959) die Greifeinrichtung ein Bestandteil des Auswertegerätes ist und das Testelement unmittelbar von der Entnahmeposition des Vorratsbehälters an das Auswertegerät übernommen wird.

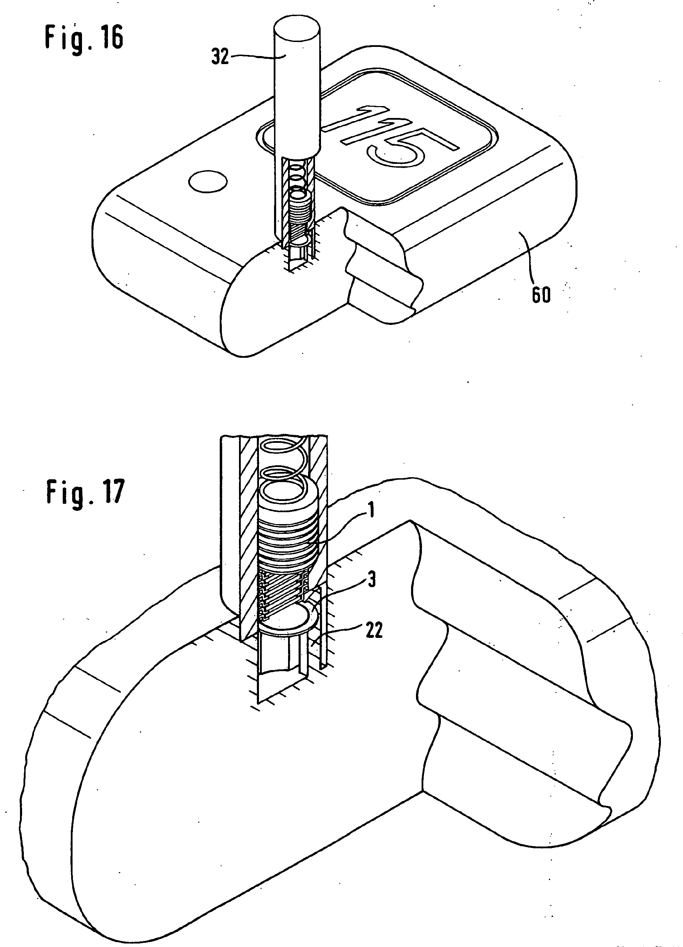

Die Figuren 15 bis 17 zeigen ein solches System, bestehend aus einem Auswertegerät 60 und einem Magazin 32. Die in den Figuren 16 und 17 erkennbare Testelementhalterung 61 schließt eine Greifeinrichtung 20 mit mehreren (z.B. drei) Greifarmen 22 ein. Die Greifarme 22 sind derartig positioniert, ausgebildet und elastisch gestaltet, daß sie jeweils ein Testelement 1 an seinem Rahmen 3 ergreifen und fixieren, wenn das Magazin 32 in der dargestellten Weise in eine Testelementöffnung 62 des Auswertegeräts 60 eingeführt wird. Wenn danach das Magazin 32 (in den Figuren nach oben) weggezogen wird, bleibt das Testelement 1 in der von den Greifarmen 22 gebildeten Halterung zurück und befindet sich in einer Position, in der es leicht mit der Probe kontaktiert werden kann. Anschließend findet die Auswertung mittels einer hier nicht dargestellten Auswerteeinrichtung statt.

Die Herstellung eines Testelementes schließt (im Falle von Testelementen, deren Testfeld getrennt von dem Rahmen hergestellt wird) folgende Verfahrensschritte ein:
- Die Rahmen 3 werden vorzugsweise durch plastisches Verformen einer Folie (vorzugsweise Kunststoffolie, jedoch kann auch eine Metallfolie verwendet werden), insbesondere durch Prägen oder Ziehen in die gewünschte Profilform gebracht.
- Zugleich oder anschließend wird erforderlichenfalls die Meßöffnung ausgestanzt, die im Fall colorimetrischer Testelemente die Auswertezone 19 umrahmt.
- Das Testfeld wird ebenfalls ausgestanzt, wobei die beschriebene Bedingung hinsichtlich dessen Durchmesser D_{f} in Relation zu der lichten Weite W des Rahmens 3 eingehalten werden sollte.
- Das Testfeld 5 wird vorteilhafterweise mittels eines Stempels, dessen Außendurchmesser kleiner als die Testfeldöffnung 2, ist aus einem Zwischenträger heraus direkt in die Aufnahmemulde 40 gedrückt.
- Schließlich werden die fertigen Testelemente aus der Folie ausgestanzt, wobei die Dimensionen des Stanzwerkzeuges die äußere Begrenzung Dₐ der Testelemente 1 bestimmen.

## Patentansprüche

1. Testelementanalysesystem zur analytischen Untersuchung von flüssigen Proben, insbesondere von Körperflüssigkeiten von Menschen oder Tieren, umfassend Testelemente (1) mit einem Testfeld (5), das zur Durchführung einer Analyse mit der Probe in Kontakt gebracht wird, wobei die Reaktion eines in der Probe enthaltenden Analyten mit mindestens einem in dem Testelement (1) enthaltenen Reagenz zu einer für die Analyse charakteristischen Änderung einer Meßgröße führt, und
ein Auswertegerät mit
einem Testelement-Vorratsbehälter (10), in dem eine Mehrzahl von Testelementen (1) zur Entnahme an einer Entnahmeposition (9) vorrätig gehalten wird,
einer Probenaufgabeposition (11), an der das Testfeld (5) jeweils eines Testelementes (1) mit der Probe (12) in Kontakt gebracht wird,
einer Transporteinrichtung (8), durch die je ein Testelement (1) an der Entnahmeposition (9) aus dem Testelement-Vorratsbehälter (10) entnommen und zu der Probenaufgabeposition (11) transportiert wird, und
einer Meßeinrichtung (14), mittels der an einem Testelement (1) die für die Analyse charakteristische Meßgröße gemessen wird
bei welchem
die Testelemente (1) einen das Testfeld (5) wenigstens teilweise umgebenden Rahmen (3) mit einem am äußeren Umfang der Testelemente (1) umlaufenden nach außen gerichteten Greifrand (24) aufweisen und
die Transporteinrichtung (8) eine Greifeinrichtung (20) zum Greifen je eines Testelementes (1) aufweist, mittels der je ein Testelement (1) auf mindestens einem Teil des Transportweges von der Entnahmeposition (9) zu der Probenaufgabeposition (11) an seinem Greifrand festgehalten wird.

2. Testelementanalysesystem nach Anspruch 1, bei welchem die Meßgröße an einer von der Probenaufgabeposition verschiedenen Meßposition (13) gemessen und das Testelement auf mindestens einem Teil des Transportweges von der Probenaufgabeposition (11) zu der Meßposition (13) mittels der Greifeinrichtung (21) festgehalten wird.

3. Testelementanalysesystem zur analytischen Untersuchung von flüssigen Proben, insbesondere von Körperflüssigkeiten von Menschen oder Tieren, umfassend Testelemente (1) mit einem Testfeld (5), das zur Durchführung einer Analyse mit der Probe in Kontakt gebracht wird, wobei die Reaktion eines in der Probe enthaltenden Analyten mit mindestens einem in dem Testelement (1) enthaltenen Reagenz zu einer für die Analyse charakteristischen Änderung einer Meßgröße führt,
einen Testelement-Vorratsbehälter (10), in dem eine Mehrzahl von Testelementen (1) zur Entnahme an einer Entnahmeposition (9) des Vorratsbehälters (10) vorrätig gehalten wird, und
ein Auswertegerät mit einer Testelement-Halterung zur Positionierung von jeweils einem Testelement in einer Probenaufgabeposition (11), an der sein Testfeld (5) mit der Probe (12) in Kontakt gebracht wird, und mit einer Meßeinrichtung (14) zum Messen der für die Analyse charakteristischen Meßgröße,
bei welchem
die Testelemente (1) einen das Testfeld (5) wenigstens teilweise umgebenden Rahmen (3) mit einem am äußeren Umfang der Testelemente (1) umlaufenden nach außen gerichteten Greifrand (24) aufweisen,
der Durchmesser des Rahmens (3) von dem Greifrand (24) aus in beiden senkrecht zu der Testfeldebene (4a,4b) verlaufenden Raumrichtungen (Z⁺ und Z⁻) zunimmt, und
das System eine Greifeinrichtung (20) einschließt, mittels der je ein Testelement (1) bei der Entnahme aus dem Vorratsbehälter (10) an seinem Greifrand gehalten wird.

4. Testelementanalysesystem nach Anspruch 3, bei welchem die Greifeinrichtung (20) ein Bestandteil des Auswertegerätes ist und das Testelement (1) unmittelbar von der Entnahmeposition des Vorratsbehälters in das Auswertegerät übernommen wird.

5. Testelementanalysesystem nach einem der vorhergehenden Ansprüche, **dadurch gekennzeichnet, daß** die Greifeinrichtung eine Mehrzahl von Greifarmen (22) einschließt, die beim Festhalten eines Testelementes (1) dessen Greifrand (24) mindestens punktweise kontaktieren.

6. Testelementanalysesystem nach Anspruch 5, bei welchem die Arme (22) der Greifeinrichtung (20) derartig elastisch beweglich sind, daß sie aufgrund der Elastizität auf das Testelement (1) geschoben werden können und das Testelement festhalten.

7. Testelementanalysesystem nach Anspruch 6, bei welchem die Arme (22) der Greifeinrichtung (20) Bestandteil eines einstückig aus einem elastischen Material hergestellten Greifelementes (26) sind.

8. Testelementanalysesystem nach einem der vorhergehenden Ansprüche, bei welchem der Testelement-Vorratsbehälter (10) ein Magazin (32) einschließt, in dem die Testelemente (1) stapelförmig übereinander gelagert sind.

9. Testelementanalysesystem nach einem der vorhergehenden Ansprüche, bei welchem die Greifeinrichtung (20) als Greifgabel (21) mit zwei Greifarmen (22) ausgebildet ist und das Testelement (1) mittels den Greifrand (24) mindestens punktweise kontaktierenden parallel zu der Testfeldebene (4) verlaufenden Greifabschnitten (25) der Arme (22) der Greifgabel (21) festgehalten wird.

10. Testelementanalysesystem nach Anspruch 9, bei welchem der Abstand zwischen den Armen (22) der Greifgabel (21) zum vorderen Ende (28) des Greifabschnittes (25) hin abnimmt.

11. Testelementanalysesystem nach Anspruch 1 und einem der Ansprüche 9 oder 10, bei welchem die Transporteinrichtung (8) so ausgebildet ist, daß jeweils ein Testelement (1) mittels einer eindimensionalen translatorischen Bewegung der Greifgabel (21) aus dem Testelement-Vorratsbehälter (10) entnommen wird.

12. Testelementanalysesystem nach Anspruch 1 und einem der Ansprüche 9 bis 11, bei welchem die Transporteinrichtung (8) so ausgebildet ist, daß jeweils ein Testelement (1) auf mindestens einem Teil des Transportweges zwischen der Entnahmeposition (9) und der Probenaufgabeposition (11) durch eine Schwenkbewegung der Greifgabel (21) um eine feste, senkrecht zu der Testfeldebene verlaufende Achse transportiert wird.

13. Testelement für ein Analysesystem zur analytischen Untersuchung von Flüssigkeiten, insbesondere für ein System nach einem der Ansprüche 1 bis 12, mit einem ein Testfeld (5) mindestens teilweise umgebenden Rahmen (3),
bei welchem
der Rahmen (3) an seinem äußeren Umfang einen nach außen gerichteten Greifrand (24) aufweist, der so ausgebildet ist, daß das Testelement (1) an dem Greifrand (24) von einer Greifeinrichtung (20) gehalten werden kann, und
der Durchmesser des Rahmens (3) von dem Greifrand (24) aus in beide Richtungen (Z⁺ und Z⁻) zunimmt.

14. Testelement nach Anspruch 13, bei welchem die Fläche des Rahmens (3) auf der Probenaufgabeseite (6) des Testelementes (1) höchstens dreimal so groß wie die Probenaufgabefläche (18) des Testfeldes (5) ist.

15. Testelement nach einem der Ansprüche 13 oder 14, dessen Dicke (d) mindestens 0,3 mm und höchstens 3 mm beträgt.

16. Testelement nach einem der Ansprüche 13 bis 15, welches in Aufsicht auf das Testfeld kreisrund ist.

17. Testelement nach einem der Ansprüche 13 bis 16, dessen Querschnittsprofil so gestaltet ist, daß mehrere übereinander gestapelte Testelemente gegeneinander in Richtung der Testfeldebene (4a,4b) gleiten können, ohne zu verhaken.

18. Testelement nach einem der Ansprüche 13 bis 17, bei welchem der Durchmesser des Rahmens (3) von dem Greifrand (24) aus in einer senkrecht zu der Testfeldebene (4a,4b) verlaufenden Raumrichtung (Z⁺) unter Ausbildung einer vorspringenden Schulter (31) zunimmt.

19. Testelement nach Anspruch 18, bei welchem die vorspringende Schulter (31) so ausgebildet ist, daß die der Schulter benachbarten Teile der Greifeinrichtung (20) von ihr abgedeckt werden.

20. Testelement nach Anspruch 19, bei welchem die Oberfläche der vorspringenden Schulter zumindest auf der Probenaufgabeseite hydrophob ist.

21. Testelement nach einem der Ansprüche 13 bis 20, bei welchem der Rahmen (3) aus Metall oder einem Kunststoffmaterial hergestellt ist.

22. Testelement nach einem der Ansprüche 13 bis 21, bei welchem der Rahmen (3) eine Aufnahmemulde (40) zur Aufnahme des Testfeldes (5) umschließt und die Tiefe der Aufnahmemulde (40) größer als die Dicke des Testfeldes (5) ist, so daß die umlaufende Begrenzungswand (38) der Aufnahmemulde (40) die Oberfläche eines darin aufgenommenen Testfelds (5) überragt.

23. Testelement nach Anspruch 22, bei welchem das Testfeld (5) ein getrennt von dem Rahmen (3) hergestelltes Teil ist, das in der Aufnahmemulde (40) fixiert ist.

24. Testelement nach Anspruch 23, bei welchem das Testfeld aus mehreren übereinander angeordneten Testschichten besteht.

25. Testelement nach einem der Ansprüche 23 oder 24, bei welchem die Begrenzungswände (38) der Aufnahmemulde (40) auf einem Teilabschnitt ihrer Höhendimension eine negative Steigung haben, so daß der Durchmesser der Aufnahmemulde (40) an deren Boden (39) größer als oberhalb ihres Bodens (39) ist und das Testfeld (5) in der Aufnahmemulde (40) **dadurch** fixiert ist, daß die lichte Weite (W) der Aufnahmemulde (40) geringer als die Außendimension (D_{f}) des Testfeldes ist, so daß das Testfeld beim Einsetzen in die Aufnahmemulde (40) radial komprimiert wird.

26. Verfahren zur Herstellung von Testelementen nach einem der Ansprüche 13 bis 25, bei welchem das Profil des Randes durch plastisches Verformen einer Kunststoff- oder Metallfolie hergestellt wird.

27. Verfahren nach Anspruch 26 zur Herstellung von Testelementen nach einem der Ansprüche 22 bis 25, welches ein Verfahrensschritt einschließt, bei dem die Aufnahmemulden (40) zur Aufnahme einer Mehrzahl von Testfeldern (5) durch Verformen einer Kunststoff- oder Metallfolie erzeugt werden.

28. Verfahren nach Anspruch 27 zur Herstellung von Analyseelementen nach einem der Ansprüche 23 bis 25, bei welchem in eine Mehrzahl von in einer Folie erzeugten Aufnahmemulden (40) Testfelder (5) eingebracht und fixiert und danach die Testelemente aus der Folie ausgestanzt werden.

29. Testelement-Vorratsbehälter, enthaltend eine Mehrzahl von Testelementen (1) nach einem der Ansprüche 13 bis 25.

30. Testelement-Vorratsbehälter nach Anspruch 29, welcher ein Magazin (32) einschließt, in dem die Testelemente (1) stapelförmig übereinander gelagert sind.

31. Testelement-Vorratsbehälter nach Anspruch 30, wobei das Magazin (32) röhrenförmig ausgebildet ist und einen parallel zu der Testfeldebene (4) von darin enthaltenen Testelementen (1) verlaufenden Entnahmeschlitz (36) aufweist, der etwas höher ist, als die Dicke (d) eines Testelementes (1), so daß jeweils ein Testelement (1) von einer Greifeinrichtung (20) durch den Entnahmeschlitz (36) aus dem Magazin (32) entnommen werden kann.

32. Testelement-Vorratsbehälter nach Anspruch 30, dessen Innenquerschnitt so auf den Außenquerschnitt der darin enthaltenen Testelemente (1) abgestimmt ist, daß die Rahmen (3) der Testelemente (1) in dichtendem Kontakt zu der Innenwand des Testelement-Vorratsbehälters (10) stehen.

## Claims

1. Test element analysis system for the analytical investigation of liquid samples, in particular of body liquids of humans or animals, comprising
test elements (1) with a test field (5), which for performing an analysis is brought into contact with the sample, the reaction of an analyte contained in the sample with at least one reagent contained in the test element (1) leading to a change of a measurable variable which is characteristic for the analysis, and
an evaluation instrument with
a test element storage container (10), where a plurality of test elements (1) are stored to be taken out at a take out position (9),
a sample application position (11), where the test field (5) of a test element (1) is brought into contact with the sample (12),
a transport device (8), for taking a test element (1) out of the test element storage container (10) at the take out position (9) and for transporting the test element to the sample application position (11), and
a measuring device (14), for measuring the measurable variable of the test element (1) which is characteristic for the analysis
wherein
the test elements (1) comprise a frame (3) at least partially surrounding the test field (5) and including an outwardly oriented gripping rim (24) running around the outer circumference of the test elements (1) and
the transport device (8) comprises a gripping device (20) for gripping a test element (1), the test element (1) being held at its gripping rim during at least a part of the transport path from the take out position (9) to the sample application position (11).

2. Test element analysis system according to claim 1, wherein the measurable variable is measured at a measuring position (13) different from the sample application position and wherein the test element is held, by means of the gripping device (21), at least during a part of the transport path from the sample application position (11) to the measuring position (13).

3. Test element analysis system for the analytical investigation of liquid samples, in particular of body liquids of humans or of animals, comprising
test elements (1) with a test field (5), which for performing an analysis is brought into contact with the sample, the reaction of an analyte contained in the sample with at least one reagent contained in the test element (1) leading to a change of a measurable variable which is characteristic for the analysis, and
a test element storage container (10), where a plurality of test elements (1) are stored to be taken out of the storage container (10) at a take out position (9), and
an evaluation instrument with a test element holder for positioning a test element in a sample application position (11), where its test field (5) is brought into contact with the sample (12), and with a measuring device (14) for measuring the change of a measurable variable which is characteristic for the analysis,
wherein
the test elements (1) comprise a frame (3) at least partially surrounding the test field (5) and including an outwardly oriented gripping rim (24) running around the outer circumference of the test elements (1),
the diameter of the frame (3) increases from the gripping rim (24) in both spatial directions (Z+ and Z-) running vertical to the test field plane (4a,4b), and
the system includes a gripping device (20), which during the taking out from the storage container (10) holds a test element (1) at its gripping rim.

4. Test element analysis system according to claim 3, wherein the gripping device (20) is a part of the evaluation instrument, and wherein the test element (1) is taken over directly from the take out position of the storage container to the evaluation unit.

5. Test element analysis system according to any one of the preceding claims, **characterized in that** the gripping device comprises a plurality of gripping arms (22), which are during the holding of the test element (1) in at least point contact with the gripping rim (24) of the test element (1).

6. Test element analysis system according to claim 5, wherein the arms (22) of the gripping device (20) are elastically moveable in such a manner that due to this elasticity they can be pushed onto the test element (1) for holding thereof.

7. Test element analysis system according to claim 6, wherein the arms (22) of the gripping device (20) are part of a gripping element (26), which is made of a single piece of an elastically deformable material.

8. Test element analysis system according to any one of the preceding claims, wherein the test element storage container (10) comprises a magazine (32) where the test elements (1) are stored in a stack one upon the other.

9. Test element analysis system according to any one of the preceding claims, wherein the gripping device (20) is embodied as a gripping fork (21) with two gripping arms (22), and wherein the test element (1) is held, by means of gripping sections (25) of the arms (22) of the gripping fork (21), the gripping sections (25) running parallel to the test field plane (4) and being in at least point contact with the gripping rim (24).

10. Test element analysis system according to claim 9, wherein the distance between the arms (22) of the gripping fork (21) decreases towards the front end (28) of the gripping section (25).

11. Test element analysis system according to claim 1 and any one of claims 9 or 10, wherein the transport device (8) is embodied in such a manner that one test element (1) at a time is taken out from the test element storage container (10) by means of a one-dimensional translatory motion of the gripping fork (21).

12. Test element analysis system according to claim 1 and any one of the claims 9 to 11, wherein the transport device (8) is embodied in such a manner that the test element (1) is transported, during at least a part of the transport path between the take out position (9) and the sample application position (11), by means of a swiveling movement of the gripping fork (21) around a fixed axis which runs vertical to the test field plane.

13. Test element for an analysis system for the analytical investigation of liquids, in particular for a system according to any one on claims 1 to 12, with a frame (3) at least partially surrounding the test field (5),
wherein
the frame (3) comprises at its outer circumference an outwardly oriented gripping rim (24) formed and arranged in such a manner that the test element (1) can be held at the gripping rim (24) by means of a gripping device (20), and
the diameter of the frame (3) increases from the gripping rim (24) in both directions (Z⁺ and Z⁻).

14. Test element according to claim 13, wherein the surface area of the frame (3) at the sample application side (6) of the test element (1) is at most three times as large as the area of the sample application surface (18) of the test field (5).

15. Test element according to any one of claims 13 or 14, having a thickness (d) of at least 0.3 mm and at most 3 mm.

16. Test element according to any one of claims 13 to 15, which is circular in top view onto the test field.

17. Test element according to any one of claims 13 to 16, having a cross sectional profile such that a plurality of test elements stacked one upon the other can slide upon another in the direction of the test field plane (4a,4b) without interlocking.

18. Test element according to any one of claims 13 to 17, wherein the diameter of the frame (3) increases from the gripping rim (24) in a spatial direction (Z⁺) vertical to the test field plane (4a,4b), forming a protruding shoulder (31).

19. Test element according to claim 18, wherein the protruding shoulder (31) is shaped and arranged in such a manner that the parts of the gripping device (20), which are adjacent to the shoulder, are covered thereby.

20. Test element according to claim 19, wherein the surface of the protruding shoulder, at least on the sample application side, is hydrophobic.

21. Test element according to any one of claims 13 to 20, wherein the frame (3) is made of metal or of a plastic material.

22. Test element according to any one of claims 13 to 21, wherein the frame (3) surrounds a reception trough (40) for receiving the test field (5), and wherein the depth of the reception trough (40) is larger than the thickness of the test field (5), so that the circumferential limiting wall (38) of the reception trough (40) extends beyond the surface of a test field (5) received thereby.

23. Test element according to claim 22, wherein the test field (5) is a part separately produced from the frame (3) and fixed in the reception trough (40).

24. Test element according to claim 23, wherein the test field comprises a plurality of test layers arranged one upon the other.

25. Test element according to any one of claims 23 or 24, wherein the limiting walls (38) of the reception trough (40) have a negative ascent at a partial section of their height dimension, so that the diameter of the reception trough (40) at its bottom (39) is larger than above its bottom (39), and the test field (5) is fixed in the reception trough (40) by the fact that the clear width (W) of the test field reception trough (40) is smaller than the outer dimension (D_{f}) of the test field, so that the test field is during insertion into the reception trough (40) slightly compressed in radial direction.

26. Method for the production of test elements according to any one of claims 13 to 25, wherein the rim profile of the frame is produced by plastic deformation of a metal foil or a plastic foil.

27. Method according to claim 26 for the production of test elements according to any one of claims 22 to 25, comprising a process step in which the reception troughs (40) for the reception of a plurality of test fields (5) are generated by deforming a plastic foil or a metal foil.

28. Method according to claim 27 for the production of analysis elements according to any one of claims 23 to 25, wherein test fields (5) are inserted and fixed in a plurality of reception troughs (40) generated in a foil and the test elements are thereafter stamped out of the foil.

29. Test element storage container containing a plurality of test elements (1) according to any one of claims 13 to 25.

30. Test element storage container according to claim 29 comprising a magazine (32) where the test elements (1) are stored in a stack one upon the other.

31. Test element storage container according to claim 30 wherein the magazine (32) is tube-shaped and comprises a take out slot (36) arranged parallel to the test field plane (4) of test elements contained therein which is a little higher than the thickness (d) of a test element (1) whereby one test element at a time can be taken out of the magazine (32) through the take out slot (36) by means of a gripping device (20).

32. Test element storage container according to claim 30 having an internal cross section which is adapted to the outer cross section of test elements (1) contained therein in such a manner that the frames (3) of the test elements (1) are in sealing contact with the inner walls of the test element storage container (10).

## Revendications

1. Système d'analyse servant à l'examen analytique d'échantillons liquides, notamment de liquides corporels d'hommes ou d'animaux, comprenant
des éléments d'analyse (1) présentant une zone d'analyse (5) qui est mise en contact avec l'échantillon pour effectuer une analyse, la réaction d'un analyte contenu dans l'échantillon avec au moins un réactif contenu dans l'élément d'analyse (1) entraînant le changement d'une grandeur de mesure carastéristique pour l'analyse et
un appareil d'évaluation avec
un contenant de stockage d'éléments d'analyse (10) dans lequel sont stockés une pluralité d'éléments d'analyse (1) destinés à être prélevés à partir d'une position de prélèvement (9),
une position d'alimentation d'échantillons (11) dans laquelle la zone d'analyse (5) d'un élément d'analyse (1) est mise en contact avec l'échantillon,
un dispositif de transport (8) servant à prélever un élement d'analyse (1) à chaque fois dans le contenant de stockage d'éléments d'analyse (10) à partir de la position de prélèvement (9) et à transporter celui-ci vers la position d'alimentation des échantillons (11) et
un dispositif de mesure (14) servant à mesurer la grandeur de mesure caractéristique de l'analyse sur un élément d'analyse (1)
dans lequel
les éléments d'analyse (1) présentent un cadre (3) qui entoure la zone d'analyse (5) au moins partiellement et qui est doté d'un bord de prise (24) faisant le pourtour des éléments d'analyse (1) et étant dirigé vers l'extérieur et
le dispositif de transport (8) présente un dispositif de prise (20) pour saisir l'élément d'analyse (1) et au moyen duquel l'élément d'analyse (1) est maintenu en son bord de prise sur au moins une partie du parcours de transport à partir de la position de prélèvement (9) jusqu'à la position d'alimentation de l'échantillon (11).

2. Système d'analyse selon la revendication 1, dans lequel la grandeur de mesure est mesurée à une position de mesure (13) différente de la position d'alimentation de l'échantillon et l'élément d'analyse est maintenu en son bord de prise par le dispositif de prise (21) sur au moins une partie du parcours de transport à partir de la position d'alimentation de l'échantillon (11) jusqu'à la position de mesure (13).

3. Système d'analyse servant à l'examen analytique d'échantillons liquides, notamment de liquides corporels d'hommes ou d'animaux, comprenant
des éléments d'analyse (1) présentant une zone d'analyse (5) qui est mise en contact avec l'échantillon pour effectuer une analyse, la réaction d'un analyte contenu dans l'échantillon avec au moins un réactif contenu dans l'élément d'analyse (1) entraînant le changement d'une grandeur de mesure carastéristique pour l'analyse,
un contenant de stockage d'éléments d'analyse (10) dans lequel sont stockés une pluralité d'éléments d'analyse (1) destinés à être prélevés à partir d'une position de prélèvement (9) du contenant (10) et
un appareil d'évaluation doté d'un support servant à positionner un élement d'analyse dans une position d'alimentation de l'échantillon (11), dans laquelle sa zone d'analyse (5) est mise en contact avec l'échantillon (12), et comprenant un dispositif de mesure (14) pour mesurer la grandeur de mesure caractéristique pour l'analyse,
dans lequel
les éléments d'analyse (1) présentent un cadre (3) qui entoure la zone d'analyse (5) au moins partiellement et qui est doté d'un bord de prise (24) faisant le pourtour des éléments d'analyse (1) et étant dirigé vers l'extérieur,
le diamètre du cadre (3) augmente à partir du bord de prise (24) dans les deux directions dans l'espace (Z⁺ et Z⁻) s'étendant perpendiculairement au plan de la zone d'analyse (4a, 4b), et
le système comprend un dispositif de prise (20) au moyen duquel l'élement d'analyse (1) est maintenu en son bord de prise lors de son prélèvement du magasin (10).

4. Système d'analyse selon la revendication 3, dans lequel le dispositif de prise (20) fait partie de l'appareil d'évaluation, et l'élément d'analyse (1) est pris dans l'appareil d'évaluation directement à partir de la position de prélèvement.

5. Système d'analyse selon l'une des revendications précédentes, **caractérisé en ce que** le dispositif de prise comprend une pluralité de bras de prise (22) qui, lorsqu'ils maintiennent un élement d'analyse (1), se trouvent en contact avec son bord de prise (24) au moins ponctuellement.

6. Système d'analyse selon la revendication 5, dans lequel les bras (22) du dispositif de prise (20) sont élastiquement mobiles, de sorte qu'on peut les faire glisser sur l'élément d'analyse (1) de par leur élasticité pour maintenir l'élément d'analyse.

7. Système d'analyse selon la revendication 6, dans lequel les bras (22) du dispositif de prise (20) font partie d'un élément de prise monobloc (26) fabriqué en un matériau élastique.

8. Système d'analyse selon l'une des revendications précédentes, dans lequel le contenant de stockage des éléments d'analyse (10) comprend un magasin (32)
dans lequel les éléments d'analyse (1) sont stockés superposés en pile.

9. Système d'analyse selon l'une des revendications précédentes, dans lequel le dispositif de prise (20) se présente sous forme de fourche de prise (21) avec deux bras de prise (22), et l'élément d'analyse (1) est maintenu par des sections de prise (25) des bras (22) de la fourche (21) s'étendant parallèlement au plan de la zone d'analyse (4) et contactant le bord de prise (24) au moins ponctuellement.

10. Système d'analyse selon la revendication 9, dans lequel l'écart entre les bras (22) de la fourche de prise (21) diminue vers l'extrémité avant (28) de la section de prise (25).

11. Système d'analyse selon la revendication 1 et l'une des revendications 9 ou 10,
dans lequel le dispositif de transport (8) est conformé de sorte qu'un élément d'analyse (1) à la fois est prélevé du contenant de stockage (10) par un mouvement translatoire unidimensionnel de la fourche de prise (21).

12. Système d'analyse selon la revendication 1 et l'une des revendications 9 à 11,
dans lequel le dispositif de transport (8) est conformé de sorte qu'un élément d'analyse (1) à la fois est transporté, sur au moins une partie du parcours de transport entre la position de prélèvement (9) et la position d'alimentation de l'échantillon (11), par un mouvement de pivotement de la fourche de prise (21) autour d'un axe fixe s'étendant perpendiculairement au plan de la zone d'analyse.

13. Elément d'analyse pour un système d'analyse servant à l'examen analytique d'échantillons liquides, en particulier pour un système selon l'une des revendications 1 à 12, comprenant un cadre (3) entourant au moins partiellement une zone d'analyse (5),
dans lequel
le cadre (3) présente sur son pourtour un bord de prise (24) dirigé vers l'extérieur et conformé de sorte que l'élément d'analyse (1) peut être maintenu sur le bord de prise (24) par un dispositif de prise (20), et
le diamètre du cadre (3) augmente à partir du bord de prise (24) dans les deux directions (Z⁺ et Z⁻).

14. Elément d'analyse selon la revendication 13, dans lequel la surface du cadre (3) côté alimentation de l'échantillon (6) de l'élément d'analyse (1) est au maximum trois fois plus grande que la surface d'alimentation de l'échantillon (18) de la zone d'analyse (5).

15. Elément d'analyse selon l'une des revendications 13 ou 14, présentant une épaisseur (d) d'au moins 0,3 mm et ne dépassant pas 3 mm.

16. Elément d'analyse selon l'une des revendications 13 à 15 présentant une forme circulaire lorsqu'on regarde la zone d'analyse par dessus.

17. Elément d'analyse selon l'une des revendications 13 à 16, dont le profil de la section transversale est conformé de sorte que plusieurs éléments d'analyse empilés les uns sur les autres peuvent glisser les uns contre les autres en direction du plan de la zone d'analyse (4a, 4b) sans s'accrocher.

18. Elément d'analyse selon l'une des revendications 13 à 17, dans lequel le diamètre du cadre (3) augmente à partir du bord de prise (24) dans une direction dans l'espace (Z⁺) s'étendant perpendiculairement au plan de la zone d'analyse (4a, 4b), en formant un épaulement saillant (31).

19. Elément d'analyse selon la revendication 18, dans lequel l'épaulement saillant (31) est conformé de sorte qu'il recouvre les parties avoisinantes du dispositif de prise (20).

20. Elément d'analyse selon la revendication 19, dans lequel la surface de l'épaulement saillant est hydrophobe au moins du côté alimentation de l'échantillon.

21. Elément d'analyse selon l'une des rvendications 13 à 20, dans lequel le cadre (3) est fabriqué en métal ou en matière plastique.

22. Elément d'analyse selon l'une des revendications 13 à 21, dans lequel le cadre (3) entoure un creux de logement (40) pour le logement de la zone d'analyse (5), et la profondeur du creux de logement (40) est supérieure à l'épaisseur de la zone d'analyse (5), de sorte que la paroi de limitation (38) du creux de logement (40) faisant le pourtour dépasse la surface d'une zone d'analyse (5) logée dans ledit creux.

23. Elément d'analyse selon la revendication 22, dans lequel la zone d'analyse (5) est une pièce fabriquée indépendamment du cadre (3) et fixée dans le creux de logement (40).

24. Elément d'analyse selon la revendication 23, dans lequel la zone d'analyse consiste en plusieurs couches d'analyse superposées.

25. Elément d'analyse selon l'une des revendications 23 ou 24, dans lequel les parois de limitation (38) du creux de logement (40) présentent une pente négative dans une section partielle de leur hauteur, de sorte que le diamètre du creux de logement (40) est plus grand en son fond (39) qu'au-dessus du fond (39) et que la zone d'analyse (5) est fixée dans le creux de réception (40) par le fait que l'ouverture (W) du creux de logement (40) est inférieure à la dimension extérieure (D_{f}) de la zone d'analyse, de sorte que la zone d'analyse est comprimée radialement lorsqu'elle est placée dans le creux de logement (40).

26. Procédé pour la préparation d'éléments d'analyse selon l'une des revendications 13 à 25, dans lequel le profil du bord est fabriqué par formage plastique d'un film plastique ou d'une feuille en métal.

27. Procédé selon la revendication 26 pour la préparation d'éléments d'analyse selon l'une des revendications 22 à 25, comprenant une étape dans laquelle les creux de logement (40) destinés à recevoir une pluralité de zones d'analyse (5) sont obtenus par formage d'un film plastique ou d'une feuille en métal.

28. Procédé selon la revendication 27 pour la préparation d'éléments d'analyse selon l'une des revendications 24 à 26, dans lequel des zones d'analyse (5) sont incorporées et fixées dans une pluralité de creux de logement (40) ménagés dans un film et, ensuite, les élément d'analyse sont découpés du film.

29. Contenant de stockage d'éléments d'analyse contenant une pluralité d'éléments d'analyse (1) selon l'une des revendications 13 à 25.

30. Contenant de stockage d'éléments d'analyse selon la revendication 29 comprenant un magasin (32) dans lequel les éléments d'analyse (1) sont stockés superposés en pile.

31. Contenant de stockage d'éléments d'analyse selon la revendication 30, dans lequel le magasin (32) se présente en forme de tube et est doté d'une fente de prélèvement (36) s'étendant parallèlement au plan de la zone d'analyse (4) des éléments d'analyse (1) qui y sont contenus, ladite fente de prélèvement étant légèrement plus haute que l'épaisseur (d) d'un élément d'analyse (1), de sorte qu'un élement d'analyse (1) à la fois peut être prélevé par un dispositif de prise (20) via la fente de prélèvement (36) dans le magasin (32).

32. Contenant de stockage d'éléments d'analyse selon la revendication 31, dont la section transversale intérieure est adaptée à la section transversale extérieure des éléments d'analyse (1) qui y sont contenus, de sorte que les cadres (3) des éléments d'analyse (1) se trouvent en contact hermétique avec la paroi intérieure du contenant de stockage d'éléments d'analyse (10).
